# EUROPEAN PATENT APPLICATION

(11) **EP 2 221 049 A2**
(43) Date of publication of application: **25.08.2010**
(21) Application number: 08849054.5
(22) Date of filing: 13.11.2008
(51) Int. Cl.: A61K 9/16, A61K 9/50, A61K 31/505

(54) **SOLID PHARMACEUTICAL COMPOSITION OF DED ANOSINE**

(30) Priority: 14.11.2007 ES 200703002
(71) Applicant: Blanver Farmoquimica, LTDA, Rua Lucia 123 Parque Sao George Cotia CEP 06708-170 Sao Paulo (BR); Fundacao Oswaldo Cruz (Fiocruz)-Instituto de Tecnoligia em Farmacos (FARMANGUINHOS), Av Brasil 4365 Manguinhos Rio de Janeiro Brasil CEP 21045-900 (BR)
(72) Inventor: TICÓ GRAU, José, Ramon, Cotia, CEP 06708-170 Sao Paulo (BR); SUÑÉ NEGRE, José, Maria, Cotia, CEP 06708-170 Sao Paulo (BR)
(74) Representative: Gislon, Gabriele
(86) International application number: PCT/IB2008/003527
(87) International publication number: WO 2009/063320

(57) **Abstract**

The present invention relates to a new solid pharmaceutical composition of didanosine comprising didanosine crystals with an average particle size comprised between 50 and 500 microns, coated by a layer of an enteric coating. The pharmaceutical composition of the invention can be prepared by means of a simple process, which comprises coating the didanosine crystals with an enteric layer. Said composition has a good stability of the active ingredient, as well as a suitable release in the conditions present in the upper part of the gastrointestinal tract. It also relates to the use of said composition for preparing solid dosage forms of didanosine for their oral administration. The invention also relates to tablets and to hard gelatin capsules containing said composition.

## Description

### Field of the Art

The present invention relates to a new sold pharmaceutical composition of didanosine which can be used for oral administration in the form of tablets or capsules.

### State of the Prior Art

Didanosine is the international nonproprietary name for the active ingredient 2',3'-dideoxyinosine having the following formula:

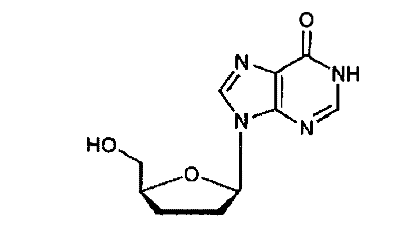

Didanosine is a synthetic nucleoside which was described for the first time in European patent application EP-A-0206497. It can also be called dideoxyinosine, ddl or ddlno.

Didanosine differs from other similar nucleosides since it does have any of the regular bases, such as for example adenine or guanine, but rather it has a hypoxanthine moiety bonded to a sugar ring.

Didanosine has antiviral activity and is used in the treatment of patients infected with the human immunodeficiency virus (HIV) in combination with other antiretroviral drugs.

Didanosine is generally available commercially in a variety of oral dosage forms, such as for example in the form of gastro-resistant capsules, dispersible chewable tablets and as powders for oral solution.

Didanosine is a white solid having a melting point comprised between 160°C and 163°C. As indicated in the article by B.D. Anderson et al., Int. J. Pharm., 1988, 45, 27-37, didanosine can be considered as an active ingredient which is relatively soluble in water since it has a water solubility of 27.3 mg/ml at a temperature of 25°C and at a pH of approximately 6.

It is also well known to the person skilled in the art that said active ingredient, like other nucleoside derivatives, is unstable in acid pH conditions. It has thus been described, for example, that 10% of didanosine is decomposed to hypoxanthine in a time period shorter than 2 minutes at a pH below 3 and at a temperature of 37°C.

The products resulting from said hydrolysis do not have antiviral activity, and therefore the pharmaceutical preparations of didanosine which are marketed must prevent the hydrolysis of the active ingredient catalyzed by the acid medium which is in the stomach.

The monograph on didanosine prepared by the World Health Organization for its inclusion in The International Pharmacopoeia describes the known and potential impurities which can be present in compositions of didanosine, among which is found firstly hypoxanthine, already mentioned.

Different alternatives for improving the stability of didanosine against acids have been developed in the state of the art. For example, the incorporation of an antacid buffer in the pharmaceutical composition.

An example of the latter case is described in European patent application EP-A-0524579. It describes chewable tablets containing a combination of magnesium hydroxide with calcium carbonate or with basic sodium and aluminium carbonate as with antacid buffering substances.

As described in the article by B. Damle et al., Br. J. Clin. Pharmacol., 2002, 54 , 255-261, the chronic administration of compositions of didanosine including antacid compound is associated with side effects such as constipation or diarrhea and can be contraindicated in patients with impaired renal function.

An alternative to the use of antacid compounds consists of using compositions which are coated with enteric compounds protecting the active ingredient during its passage through the acid medium of the acid.

Few technical solutions relating to enteric coated compositions of didanosine have been described in the state of the art.

For example, United States patent US5225202 describes enteric coated pharmaceutical compositions containing:
a) a core, preferably in the form of a pellet, comprising:
   i. an active ingredient sensitive to the acid medium, which can be didanosine among others.
   ii. a disintegrant, and
   iii. a buffering agent to provide an additional gastric protection, and
b) an enteric layer on said nucleus comprising:
   i. the hydroxypropyl methylcellulose phthalate polymer and
   ii. a plasticizer.

Patent application PCT WO-A-99/61002 describes a pharmaceutical composition with didanosine in the form of capsules containing small enteric coated beadlets. Said beadlets are obtained by a complex process including the following steps:
a) granulating in wet conditions the active ingredient with disintegrant agents such as sodium carboxymethylcellulose and sodium starch glycolate,
b) extruding the wet mass,
c) spheronizing the extruded mass to form the small beadlets, wherein during said step a dry mixture of didanosine with suitable excipients is dusted,
d) drying the beadlets,
e) applying the enteric layer on the small beadlets in a fluid bed processor, and
f) treating the beadlets with anti-adherent agents such as talc before introducing them in the capsules.

Patent application PCT WO-A-00/03696-Al describes enteric coated didanosine tablets comprising:
a) a corp in the form of a tablet comprising:
   i. didanosine as the active ingredient,
   ii. a binding agent or a diluent,
   iii. a disintegrant agent, and
   iv. a lubricating agent, and
b) an enteric coating around said nucleus comprising:
   i. a copolymer of methacrylic acid, and
   ii. a plasticizer.

Patent application PCT WO-A-2006/054175 describes solid dosage forms of didanosine comprising:
a) a core containing the active ingredient, sodium starch glycolate and sodium carboxymethylcellulose, talc,
b) a protective layer of hydroxypropyl methylcellulose deposited on the core, and
c) an enteric layer
   In the already mentioned article by Damle *et al.,* the pharmacokinetic data of two enteric coated compositions, mini-tablets and beadlets are compared with that of the chewable tablets containing antacid substances. From the results of said study it is concluded that:
   - the enteric compositions lead to substantially lower maximum plasma levels (C_{MAX}), approximately 40%, than those obtained with the chewable tablets
   - the time for obtaining the maximum concentration (t_{MAX}) goes from approximately 0.67 hours for the chewable tablets to 1.33 hours for the enteric beadlets, and to 2.83 hours for the enteric tablets, and
   - the area under the curve representing the plasma concentration against time (AUC) is equivalent for all the formulations tested.

It is therefore observed that the enteric compositions described in the state of the art are an alternative for the chronic administration of didanosine. However, is also evident that the enteric dosage forms described in the state of the art have drawbacks for a satisfactory industrial implementation, since for the preparation thereof the use of processes consisting of multiple phases, generally in different pieces of equipment, is required, with the consequent handling of multiple products and the loss of yield, as well as the possible generation of impurities due to the decomposition of the active ingredient throughout the production process.

It is therefore still necessary to have alternative solid pharmaceutical compositions of didanosine which can be prepared by means of simpler processes, maintaining a suitable stability and release of the active ingredient.

### Object of the Invention

The authors of this invention have discovered a new solid pharmaceutical composition of didanosine which can be prepared by means of a simpler process than those described in the state of the art and which furthermore has a good stability and a suitable release of the active ingredient.

The object of the invention is a solid pharmaceutical composition of didanosine.

A process for preparing the mentioned composition forms part of the object of the invention.

The pharmaceutical composition obtainable by means of the process of the invention also forms part of the object of the invention.

The use of the pharmaceutical composition for preparing solid forms of didanosine for their oral administration likewise forms part of the object of the invention.

A hard gelatin capsule comprising the mentioned composition also forms part of the object of the invention.

A didanosine tablet comprising the mentioned composition also forms part of the object of the invention.

### Detailed Description of the Invention

The object of the invention is a solid pharmaceutical composition of didanosine comprising:
a) a core consisting of didanosine crystals with an average particle size comprised between 50 and 500 microns, and
b) an enteric layer on said core comprising an enteric polymer.

The authors of this invention have developed a new pharmaceutical composition of didanosine in which the enteric layer is directly applied on the active ingredient crystals, thus reducing the handling of products and minimizing the number of pieces of industrial equipment necessary for its manufacture.

### The active ingredient

The active ingredient didanosine can be prepared according the process described in patent application EP-A-206497.

In the composition of the invention, the didanosine crystals can have any crystalline form thereof, including polymorphs, such as for example the polymorphs described in the article by R. Bettini *et al.* presented at the 2007 AAPS Annual Meeting & Exposition in San Diego (USA).

The average size of the didanosine crystals is comprised between 50 and 500 microns, preferably between 100 and 350 microns, more preferably between 150 and 300 microns and even more preferably between 200 and 250 microns.

In the context of the invention, the average size of the crystals relates to the average size by volume as described in chapter 3 of the book F.A. Zenz, D.F. Othmerm Fluidization and Fluid Particle Systems, Reinhold Publishing Corp. 1960. Said parameter is one of the parameters usually provided by the pieces of equipment used for determining the particle size such as for example the piece of equipment from the COULTER COUNTER® LS series of the Beckman Coulter company.

### The enteric layer

The enteric layer can also be called enteric film or enteric coating, and it is that costing which remains intact in the stomach, but which dissolves once it reaches the small intestine and releases the content of the dosage form. It also usually called gastro-resistant coating because it can cross the stomach in a substantially unchanged manner.

The purpose of an enteric layer is therefore to delay the release of active ingredients which are inactivated by the content of the stomach, for example didanosine, or which can cause nausea or bleeding due to the irritation of the gastric mucosa, for example aspirin or steroids among others.

The action of enteric coatings is the result of the difference in the composition of the stomach environment and of the intestinal environment in relation to the pH and to enzymatic properties. The enteric coatings which are normally used thus remain unchanged in the pH conditions of the stomach, and on the contrary they dissolve rapidly when the pH increases to values greater than 4, which is the pH in the intestine.

The enteric layer comprises an enteric polymer.

The enteric polymer can be selected, for example, from the group consisting of: cellulose acetate phthalate, hydroxypropyl methylcellulose phthalate, polyvinyl acetate phthalate, copolymers of methacrylic acid and acrylic or methacrylic acid esters and/or mixtures thereof. The enteric polymer is preferably a copolymer of methacrylic acid and acrylic or methacrylic acid esters, such as Eudragit^{®} L-30 D-55, Eudragit^{®} L12-5, Eudragit^{®} L100.

For example, the enteric polymer Eudragit® L-30 D-55 is a copolymer of methacrylic acid and ethyl acrylate in a 1:1 molar ratio which is soluble in water. Eudragit^{®} L12-5 and Eudragit^{®} L100 are copolymers of methacrylic acid and methyl methacrylate in a 1:1 molar ratio soluble in acetone and alcohols.

In the composition of the invention an enteric polymer selected from the group consisting of copolymers of methacrylic acid and ethyl acrylate in a 1:1 molar ratio, and copolymers of methacrylic acid and methyl methacrylate in a 1:1 molar ratio is preferably used.

Enteric polymers based on copolymers of methacrylic acid and acrylic acid or methacrylic acid esters can be combined with copolymers of acrylic acid esters and methacrylic acid esters, such as for example copolymers from the Eudragit^{®} NE series and from the Eudragit^{®} NM series. Eudragit^{®} NE-30D, Eudragit^{®} NE-40D, and Eudragit^{®} NM-30D, which are copolymers of ethyl acrylate and methyl methacrylate, can be mentioned among them.

In a preferred embodiment, the enteric layer is formed by a mixture of a copolymer of methacrylate acid and ethyl acrylate in a 1:1 molar ratio and a copolymer of ethyl acrylate and methyl methacrylate.

An example of said preferred embodiment is the combination of the enteric polymers Eudragit^{®} L-30 D-55 and Eudragit^{®} NE-30D.

The polymers used to prepare enteric layers are generally soluble or dispersible in acetone, alcohols or in water, such that solutions or dispersions thereof in said solvents can be prepared for their application.

The amount of enteric polymer which is normally applied is comprised between 10% and 50% by weight over the weight of didanosine, preferably between 15% and 35%, and even more preferably between 28% and 22%.

The enteric layer can further contain auxiliary agents for modifying the physical characteristics thereof, for improving the chemical compatibility with the active ingredient, and/or for incorporating aesthetic attributes to the formulation of the invention.

Plasticizers, alkaline compounds, pigments and/or colorants are among the auxiliary agents which can be used.

A plasticizer is a substance which is usually used to improve the mechanical properties of the film formed by a polymeric type substance.

Plasticizers are generally incorporated to the enteric layer to regulate the physical properties of the film, and confer flexibility thereto in order to be adapted to the didanosine crystals which are coated.

The plasticizer can be selected, for example, from: polyethylene glycol, propylene glycol, glycerin, simethicone, triacetin, citric acid esters such as tributyl citrate, sebacic acid esters such as dibutyl sebacate, phthalic acid esters such as dimethyl phthalate, medium chain fatty acid mono-, di- and triglycerides, castor oil, long chain fatty acids and/or mixtures thereof.

The plasticizer is preferably selected from the group consisting of triethyl citrate, dimethyl phthalate and dibutyl sebacate, and more preferably the plasticizer is triethyl citrate.

The plasticizer content can be comprised between 3% and 30% by weight over the weight of the enteric polymer, and more typically between 10% and 25%.

In the event of using enteric polymers which are formed by acid monomers, such as for example acrylic acid or methacrylic acid, the enteric layer generally contains alkaline compounds to neutralize said acidity, such as for example sodium hydroxide, tromethamine, triethanolamine, arginine or lysine. Preferably an alkaline compound is used selected from the group consisting of sodium hydroxide, tromethamine, arginine and lysine, more preferably it is selected from the group consisting of sodium hydroxide and tromethamine.

The enteric layer can also contain pigments or colorants to modify the degree of opacity thereof and/or to confer aesthetic attributes to the formulation of the invention. The following can be mentioned among suitable pigments and colorants: talc, titanium dioxide, calcium carbonate, zinc oxide, iron oxides, alumina, chromium oxide green, cochineal extract, iron ferrocyanide, mica, pyrogallol, copper powder, bronze powder, bismuth oxychloride, beta-carotene, tartrazine, brilliant blue FCF, or erythrosine.

The pigment is preferably selected from the group consisting of talc and titanium dioxide.

The enteric layer can be applied by using pieces of equipment well known to the person skilled in the art and which are described, for example, in the book about pharmaceutical technology: Remington, The Science and Practice of Pharmacy, 20th Edition, Philadelphia, Lippincott, Williams & Wilkinson, 2000 [ISBN 0 683 606472]. Different fluid bed processor models can be mentioned among them. For example:
- the granulator type in which the spraying of the solution or dispersion containing the enteric polymer and possibly the auxiliary agents on the solid particles takes place from the upper part of the piece of equipment,
- the Wurster type in which the spraying takes place from the lower part, and
- the rotor type, in which the spraying is tangential.

The composition of the invention can contain agglomerates of crystals coated with the enteric layer which can be formed during the manufacturing process by adhesion between the didanosine crystals brought about by the enteric layer which has not completely dried. The case that inside the agglomerates there are crystals which are not completely coated by an enteric layer may also occur, although the agglomerates as such are substantially coated with an enteric layer.

### The insulating layer

In a preferred embodiment the composition of the invention comprises an insulating layer between the didanosine crystals and the enteric layer, wherein said insulating layer comprises a film-forming agent.

In this case, the insulating layer is directly applied on didanosine crystals by using a piece of equipment such as those mentioned in the already mentioned Remington book, for example a Wurster type fluid bed processor. Once the insulating layer has been applied, the coated didanosine crystals are dried in the same piece of equipment.

The insulating layer is applied on didanosine crystals by using solutions or dispersions in water or organic solvents of a film-forming agent.

The film-forming agent is generally an inert polymeric compound such as for example, polyethylene glycol, polyvinyl alcohol, methylcellulose, hydroxypropyl methylcellulose, hydroxypropyl cellulose, ethyl cellulose, polyvinylpyrrolidone and/or mixtures thereof.

In the compositions of the invention, the film-forming agent is preferably selected from the group consisting of hydroxypropyl methylcellulose, hydroxypropyl cellulose and ethyl cellulose, more preferably it is hydroxypropyl methylcellulose.

For the purpose of increasing the regulatory properties of the insulating layer, said insulating layer can possibly further contain auxiliary agents such as for example alkaline compounds with antacid properties.

Magnesium oxide, magnesium hydroxide, magnesium carbonate, sodium hydroxide, sodium carbonate, tromethamine, triethanolamine, arginine, lysine and/or mixtures thereof, for example, can be mentioned among the alkaline compounds.

The insulating layer is generally applied in an amount comprised between 5 and 15 mg/cm², and generally its thickness is not less than 2 microns.

### Preparation process

The process for preparing the solid pharmaceutical composition of the invention is substantially simpler than the processes described for preparing enteric oral forms of didanosine.

The process for preparing the composition of the invention comprises:
a) placing the didanosine crystals in a fluid bed processor and keeping them at a temperature comprised between 20°C and 30°C,
b) applying an enteric layer comprising an enteric polymer, and
c) drying the didanosine crystals coated with the enteric layer.

The enteric layer can be used by applying a solution or dispersion of an enteric polymer in water or in a pharmaceutically acceptable organic solvent. As has already been mentioned above, the enteric layer can further comprise auxiliary agents for modifying the physical characteristics thereof, for improving the chemical compatibility with the active ingredient, and/or for incorporating aesthetic attributes to the formulation of the invention.

The following can be mentioned, for example, among the enteric polymers than can be used, cellulose acetate phthalate, hydroxypropyl methylcellulose phthalate, polyvinyl acetate phthalate, copolymers of methacrylic acid and acrylic or methacrylic acid esters, such as Eudragit^{®} L-30 D-55, Eudragit^{®} L12-5, Eudragit^{®} L100 and/or mixtures thereof.

As has already been mentioned above, the enteric polymers based on copolymers of methacrylic acid and acrylic or methacrylic acid esters can be combined with copolymers of acrylic acid esters and methacrylic acid esters.

Among the auxiliary agents that can be used are plasticizers, alkaline compounds, pigments and/or colorants.

In the process of the invention, it is preferable to add a lubricating agent, such as for example magnesium stearate, or a gliding agent, such as for example silicon dioxide, to facilitate the suspension of the didanosine crystals in the fluid bed processor. The amount of said agent is generally comprised between 0.2% and 0.5% by weight over the weight of the didanosine crystals.

It is well known to the person skilled in the art that to increase the thickness of the enteric layer the amount of solution or dispersion of the enteric substance to be applied can be increased, or steps b) and c) can be repeated as many times as considered necessary.

The crystals coated with the enteric layer are generally dried for 2 hours at a temperature of 40°C or for 24 hours at room temperature.

In a preferred embodiment, the process of the invention comprises the following steps:
a) placing the didanosine crystals in a fluid bed processor and keeping them at a temperature comprised between 20°C and 30°C,
b) applying an insulating layer comprising a film-forming agent,
c) drying the didanosine crystals coated with the insulating layer,
d) applying an enteric layer comprising an enteric coating, and
e) drying the didanosine crystals coated with the enteric layer.

Also in this case, the thickness of the insulating layer and of the enteric layer can be adjusted by repeating steps b) and c) and d) and e), respectively.

The crystals coated with the enteric layer can be dried under the already mentioned conditions.

Ry way of example, a process which can be used to prepare said composition is described below.

The didanosine crystals are placed in a Wurster type fluid bed processor, for example UNIGLATT or GLATT GPCG model of the Glatt company (Switzerland) or the AEROMATIC MULTIPROCESSOR of the GEA company (Germany) or the HÜTTLIN HKC5 model of the Hüttlin company (Germany), and are kept at a temperature comprised between 20°C and 30°C.

A concentrated solution or dispersion of an enteric polymer in water or in an organic solvent, which possibly contain plasticizers to regulate the properties of the film, and pigments to modify the degree of opacity thereof, are then sprayed on them. In the event of using polymers which are formed by acid monomers, such as for example acrylic or methacrylic acid, an alkaline substance which can be of organic type such as tromethamine, arginine or lysine, is generally used.

Once the crystals have been coated with the enteric layer, they can be dried for 2 hours at 40°C or for 24 hours at room temperature. Finally, if it is considered suitable, they can be separated into fractions of different size by sieving.

The composition of the invention thus obtained can be mixed with suitable excipients according to conventional techniques and metered in capsule-forming machines to prepare hard gelatin capsules, or compressed in rotary compressing machines. In each case, the suitable excipients are used, which are well known to the person skilled in the art.

In the already mentioned Remington book about pharmaceutical technology, there is abundant information about capsule-forming machines and machines for producing tablets.

In the event of applying an additional insulating layer on the didanosine crystals before applying the enteric layer, the following process can be followed, for example.

The didanosine crystals are placed in a Wurster type fluid bed processor and are kept at a temperature comprised between 20°C and 30°C.

A concentration solution or dispersion of a film-forming agent in water or in an organic solvent is then sprayed.

The film-forming agent is generally an inert polymeric compound such as for example polyethylene glycol, polyvinyl alcohol, methylcellulose, hydroxypropyl methylcellulose, hydroxypropyl cellulose, ethyl cellulose, polyvinylpyrrolidone and/or mixtures thereof.

For the purpose of increasing the regulatory properties of the insulating layer, said solution or dispersion can possibly contain substances with antacid properties such as for example inorganic compounds: magnesium oxide, magnesium hydroxide, magnesium carbonate, sodium hydroxide, sodium carbonate, or organic compounds: tromethamine, arginine or lysine.

Once the crystals coated with the insulating layer have been dried, the enteric layer is incorporated according to the already mentioned process.

The coated didanosine crystals thus obtained have a larger grain size then that of the didanosine crystals which have been used to prepare them. For example, in the event of using didanosine crystals with an average size comprised between 100 and 350 microns, the coated didanosine crystals have an average size comprised between 450 and 800 microns.

The composition of the invention thus obtained can be mixed with suitable excipients according to conventional techniques and metered in capsule-forming machines to prepare hard gelatin capsules, or compressed in rotary compressing machines.

The solid pharmaceutical composition obtainable by means of the process of the invention also forms part of the object of the invention.

### Use of the compositions

The use of the compositions of the invention for preparing solid dosage forms of didanosine for their oral administration, such as for example hard gelatin capsules, tablets or sachets for oral solution forms part of the object of the invention. Hard gelatin capsules and tablets are preferred among them.

A hard gelatin capsule comprising an amount of the solid composition of the invention which is sufficient for providing an effective unit dose of didanosine also forms part of the object of the invention.

The effective unit dose of didanosine is generally between 100 and 600 mg, preferably between 200 and 500 mg, and even more preferably between 300 and 400 mg.

The composition of the invention can be mixed with an auxiliary agent such as for example anti-adherent or gliding agents such as silicon dioxide or talc to facilitate handling. It can be then metered into hard gelatin capsules by means of using capsule-forming machines such as those described in the Remington book mentioned above.

The capsules of the invention comprise a composition of the invention containing didanosine crystals directly coated with an enteric layer. In a preferred embodiment, the capsules are filled with a composition containing didanosine crystals coated with an insulating layer located between the didanosine crystals and the enteric layer.

In a preferred embodiment, the hard gelatin capsule contains a composition of the invention comprising didanosine crystals directly coated by an enteric layer and having the following composition:
- a core consisting of didanosine crystals, and
- an enteric layer comprising a copolymer of methacrylic acid and ethyl acrylate, triethyl citrate and tromethamine.

In a more preferred embodiment, the hard gelatin capsule contains a composition comprising didanosine crystals further having an insulating layer between the core formed by didanosine crystals and the enteric layer, and having the following composition:
- a core consisting of didanosine crystals,
- an insulating layer comprising hydroxypropyl methylcellulose, tromethamine and magnesium carbonate, and
- an enteric layer comprising a copolymer of methacrylic acid and ethyl acrylate, triethyl citrate and tromethamine.

In a particularly preferred embodiment, the hard gelatin capsule contains a composition of the invention formed by didanosine crystals directly coated by an enteric layer and having the following composition:

| | **Component** | **Amount (mg)** |
|---|---|---|
| Core | Didanosine | 400 |
| Enteric layer | Copolymer of methacrylic acid | 280 |
| | and ethyl acrylate in a | |
| | 1:1 molar ratio | |
| | (Eudragit^{®} L-30_D55) | |
| | Triethyl citrate | 20 |
| | Tromethamine | 15 |
| | Talc | 10 |
| | Titanium dioxide | 5 |

In an even more preferred embodiment, the hard gelatin capsule contains didanosine crystals further having an enteric layer between the core formed by didanosine crystals and the enteric layer, wherein the insulating layer has the following composition:

| | **Component** | **Amount (mg)** |
|---|---|---|
| Insulating layer | Hydroxypropyl methylcellulose | 50 |
| | Tromethamine | 15 |
| | Magnesium carbonate | 5 |

A tablet comprising an amount of the solid composition of the invention which is sufficient for providing an effective unit dose of didanosine and at least one auxiliary agent also forms part of the object of the invention.

The effective unit dose of didanosine is generally between 100 and 600 mg, preferably between 200 and 500 mg, and even more preferably between 300 and 400 mg.

The tablets of the invention contain didanosine crystals directly coated with an enteric layer. In a preferred embodiment, the didanosine crystals are coated with an insulating layer located between the didanosine crystals and the enteric layer.

Shock absorbers, diluents, disintegrants, lubricants, anti-adherent agents, etc. can be mentioned among the suitable auxiliary agents or excipients for preparing the tablets containing the matrices of the invention.

Shock absorbing agents are excipients which are used as inter-particle mixture to prevent the enteric layer coating the crystals from breaking during the compression process. Cellulose and its derivatives, starches and gums (for example gum arabic) can be mentioned as shock absorbing agents. Gum arabic powder is preferably used.

Diluents are excipients facilitating the compression of powder materials and providing resistance to the tablets. Microcrystalline cellulose, lactose, dicalcium phosphate, PVP (polyvinylpyrrolidone), hydroxypropyl cellulose (HPC), pregelatinized starch, dry flowing starch and mixtures thereof, for example, can be used as suitable diluents. Preferred diluents for the purposes of the present invention are microcrystalline cellulose (for example, MICROCEL^{®} or AVICEL^{®}, lactose monohydrate, dicalcium phosphate (anhydrous or dihydrate), lactose/PVP mixtures (for example, LUDIPRES^{®}) or microcrystalline cellulose and lactose mixtures (for example, CELLACTOSE^{®}).

Disintegrants are excipients causing a rapid breaking of the tablet when it is introduced in aqueous medium and also a rapid breakdown of the granules, such that the active ingredient is released rapidly. Different types of starch and possibly cross-linked energetic disintegrants such as crospovidone, croscarmellose sodium, sodium starch glycolate and polymers derived from acrylic acid can be mentioned among disintegrants.

Lubricants and anti-adherent agents are excipients reducing inter-particle stresses and favoring the formation of the tablet, and they also prevent the adhesion of the particles. Talc, stearic acid alkaline earth salts, especially magnesium and calcium stearate, stearic acid, PEG 4000 and 6000 and stearyl fumarate can be mentioned among them. One of the most used anti-adherent agents is colloidal silica.

In consultation handbooks accessible for the person skilled in the art such as the book Handbook of Pharmaceutical Excipients, 4th Edition, London, Pharmaceutical Press, 2003 [ISBN 0 85369 475 9], there is information about the characteristics of the described excipients, and in most cases there also examples of the trade names under which said products can be found on the market.

In a preferred embodiment, the tablet comprises a composition of the invention comprising didanosine crystals coated directly by an enteric layer and having the following composition:
- a core consisting of didanosine crystals, and
- an enteric layer comprising a copolymer of methacrylic acid and ethyl acrylate, triethyl citrate and tromethamine.

In a more preferred embodiment, the tablet comprises a composition comprising didanosine crystals which further have an insulating layer between the core formed by didanosine crystals and the enteric layer, and having the following composition:
- a core consisting of didanosine crystals.
- an insulating layer comprising hydroxypropyl methylcellulose, tromethamine and magnesium carbonate, and
- an enteric layer comprising a copolymer of methacrylic acid and ethyl acrylate, triethyl citrate and tromethamine.

In a particularly preferred embodiment, the tablet contains didanosine crystals directly coated by an enteric layer and has the following composition:

| | **Component** | **Amount (mg)** |
|---|---|---|
| Core | Didanosine | 400 |
| Enteric layer | Copolymer of methacrylic acid | 280 |
| | and ethyl acrylate | |
| | in a 1:1 molar ratio | |
| | (Eudragit^{®} L-30-D55) | |
| | Triethyl citrate | 20 |
| | Tromethamine | 15 |
| | Talc | 10 |
| | Titanium dioxide | 5 |
| Auxiliary agents | Rice starch | 15 |
| | Gum Arabic | 50 |
| | Croscarmellose sodium | 10 |
| | Magnesium stearate | 5 |

The auxiliary agents are mixed with the enteric coated didanosine crystals before passing to the compressing machine.

In an even more preferred embodiment, the tablet contains didanosine crystals which further have an insulating layer between the core formed by didanosine crystals and the enteric layer, wherein the insulating layer has the following composition:

| | **Component** | **Amount (mg)** |
|---|---|---|
| Insulating layer | Hydroxypropyl methylcellulose | 50 |
| | Tromethamine | 15 |
| | Magnesium carbonate | 5 |

### Stability and gastro-resistance tests

Stability tests have been conducted with a composition of the invention which comprised enteric coated didanosine crystals. The stability conditions have been: a temperature of 25°C and under a relative humidity of 60%. The enteric coated matrix beadlets present in the hard gelatin capsules of the medicinal product VIDEX were tested in the same conditions.

It has surprisingly been observed that in the stability test the composition of the invention generates a substantially smaller amount of impurities than said matrix beadlets.

After 6 months, the impurity content in the composition of the invention has increased by 7.7.%, whereas in the matrix beadlets it has increased by 16.3%.

Gastro-resistance tests have also been conducted with hard gelatin capsules which contained a formulation of the invention which comprised enteric coated didanosine crystals and with the hard gelatin capsules of the medicinal product VIDEX according to the process described in the US Pharmacopoeia, using apparatus 1 or apparatus 2 and at a speed of 100 rpm at pH 1.5, corresponding to the gastric pH, and at pH 6.8 of the phosphate buffer solution, corresponding to the pH of the intestine. Samples were taken out periodically, and the didanosine content was analyzed spectrophotometrically.

It has been observed that the composition of the invention comprising enteric coated didanosine crystals has a behavior substantially equivalent to that of the matrix beadlets.

After two hours in acid medium, the percentage of didanosine released in both samples is nil, whereas in a medium buffered at pH 6.8, a percentage of didanosine greater than 90% is released in both cases, 91% in the case of the composition of the invention and 93% in the case of the matrix beadlets.

Therefore, the composition of the invention has a didanosine release behavior similar to that of the medicinal product VIDEX, in spite of having a substantially simpler structure.

The process for preparing the composition furthermore has the advantage that it is simple, since it only requires the use of a fluid bed processor, which facilitates the industrial implementation and scaling thereof.

The following examples serve to illustrate but not to limit the invention.

### Examples

### Example 1. Preparation of a composition according to the invention with an insulating layer and an enteric layer

400 g of crystalline didanosine with an average particle size of 90 microns were placed in a Wurster type fluid bed processor and were kept a temperature comprised between 20°C and 30°C.

A concentrated aqueous dispersion which contained 50 g of hydroxypropyl methylcellulose, 5 g of magnesium carbonate and 15 g of tromethamine was prepared, and with said dispersion the didanosine crystals were coated in the Wurster type fluid bed processor applying the insulating layer according to the conventional process.

The film-forming dispersion was applied slowly, due to the solubility of didanosine in water.

Once the application of the insulating layer had ended, the coated crystals were dried in the same fluid bed processor at a temperature of 40°C for 2 hours.

An aqueous dispersion was then prepared which contained: 280 g of Eudragit L-30 D55^{®}, 20 g of triethyl citrate, 15 g of tromethamine, 10 g of talc and 5 g of titanium dioxide.

Said aqueous dispersion was then applied on the didanosine crystals coated with the insulating layer in a Wurster type fluid bed according to the conventional process, and with the following conditions:

| | |
|---|---|
| Inlet air temperature | 35-55°C |
| Outlet air temperature | 25-45°C |
| Spraying pressure | 1-2 bars |
| Application pump speed | 1.3 to 1.7. |
| Air outlet gate position | 50 |
| Product temperature | 20°C-30°C |
| Air inflow | 45-90 m³/h |

Once the application of the enteric layer had ended, the coated crystals were applied in the same fluid bed processor.

Approximately 640 g of enteric coated crystals were obtained.

Example 1 was repeated and a good repeatability of the results was observed.

### Examples 2 to 17. Preparation of other compositions according to the invention with an insulating layer and an enteric layer

Compositions of didanosine according to the invention, which had different compositions of the insulating layer and of the enteric layer, as shown in Tables I to IV, were prepared following methods similar to that described in Example 1:

**TABLE I**

| **Component** | **2** | **3** | **4** | **5** |
|---|---|---|---|---|
| Didanosine | 400 | 400 | 400 | 400 |
| MgCO₃ | 5 | 5 | 5 | 5 |
| Tromethamine | 15 | 15 | - | - |
| Lysine | - | - | 15 | 15 |
| Arginine | - | - | - | - |
| HPMC | - | - | 50 | - |
| PVP | 50 | - | - | 50 |
| PEG 6000 | - | 50 | - | - |
| Eudragit^{®} L-30 D55 | 280 | 280 | 280 | 280 |
| Triethyl citrate | 20 | 20 | 20 | 20 |
| Tromethamine | 15 | 15 | 15 | 15 |
| Talc | 10 | 10 | 10 | 10 |
| Titanium dioxide | 5 | 5 | 5 | 5 |

**TABLE II**

| **Component** | **6** | **7** | **8** | **9** |
|---|---|---|---|---|
| Didanosine | 400 | 400 | 400 | 400 |
| MgCO₃ | 5 | 5 | 5 | 5 |
| Tromethamine | - | - | - | - |
| Lysine | 15 | - | - | - |
| Arginine | - | 15 | 15 | 15 |
| HPMC | - | - | - | 50 |
| PVP | - | - | 50 | - |
| PEG 6000 | 50 | 50 | - | - |
| Eudragit^{®} L-30 D55 | 280 | 280 | 280 | 280 |
| Triethyl citrate | 20 | 20 | 20 | 20 |
| Tromethamine | 15 | 15 | 15 | 15 |
| Talc | 10 | 10 | 10 | 10 |
| Titanium dioxide | 5 | 5 | 5 | 5 |

**TABLE III**

| **Component** | **10** | **11** | **12** | **13** |
|---|---|---|---|---|
| Didanosine | 400 | 400 | 400 | 400 |
| MgCO₃ | 5 | 5 | 5 | 5 |
| Tromethamine | 15 | 15 | 15 | 15 |
| HPMC | 50 | 50 | 50 | 50 |
| Eudragit^{®} L-30 D55 | 280 | 280 | 280 | 280 |
| Triethyl citrate | - | - | - | - |
| Dibutyl sebacate | 20 | - | 20 | - |
| Dimethyl phthalate | - | 20 | - | 20 |
| Tromethamine | 15 | 15 | - | - |
| Arginine | - | - | 15 | 15 |
| Talc | 10 | 10 | 10 | 10 |
| Titanium dioxide | 5 | 5 | 5 | 5 |

**TABLE IV**

| **Component** | **14** | **15** | **16** | **17** |
|---|---|---|---|---|
| Didanosine | 400 | 400 | 400 | 400 |
| MgCO₃ | 5 | 5 | 5 | 5 |
| Tromethamine | 15 | 15 | 15 | 15 |
| HPMC | 50 | 50 | 50 | 50 |
| Eudragit^{®} L-30 D55 | 280 | 280 | 280 | 280 |
| Triethyl citrate | - | 20 | - | 20 |
| Dibutyl sebacate | 20 | - | - | - |
| Dimethyl phthalate | - | 20 | - | - |
| Tromethamine | 15 | 15 | 15 | - |
| Arginine | - | - | - | 15 |
| Talc | 15 | 15 | 15 | 15 |
| Titanium dioxide | 5 | - | - | - |

Wherein the amounts are expressed in g, and HPMC means hydroxypropyl methylcellulose, PVP means polyvinylpyrrolidone and PEG means polyethylene glycol.

### Example 18. Preparation of a composition according to the invention with an enteric layer and without an insulating layer

400 g of crystalline didanosine with an average particle size of 90 microns were placed in a Wurster type fluid bed processor and were kept a temperature comprised between 20°C and 30°C.

An aqueous dispersion was then prepared which contained: 280 g of Eudragit L-30 D55^{®}, 20 g of triethyl citrate, 15 g of tromethamine, 10 g of talc and 5 g of titanium dioxide.

Said aqueous dispersion was then applied on the didanosine crystals coated with the insulating layer in a Wurster type fluid bed according to the conventional process, and with the following conditions:

| | |
|---|---|
| Inlet air temperature | 35-55°C |
| Outlet air temperature. | 25-45°C |
| Spraying pressure | 1-2 bars |
| Application pump speed | 1.3 to 1.7 |
| Air outlet gate position | 50 |
| Product temperature | 20°C-30°C |
| Air inflow | 45-90 m³/h |

Once the application of the enteric layer had ended, the coated crystals were applied in the same fluid bed processor.

Approximately 610 g of enteric coated crystals were obtained.

Example 18 was repeated and a good repeatability of the results was observed.

### Examples 19 to 26. Preparation of other compositions according to the invention with an enteric layer and without an insulating layer

Compositions of didanosine according to the invention, which had different compositions of the enteric layer, as shown in Tables V to VI, were prepared following methods similar to that described in Example 18:

**TABLE V**

| **Component** | **19** | **20** | **21** | **22** |
|---|---|---|---|---|
| Didanosine | 400 | 400 | 400 | 400 |
| Eudragit^{®} L-30 D55 | 280 | 280 | 280 | 280 |
| Triethyl citrate | - | - | - | - |
| Dibutyl sebacate | 20 | - | 20 | - |
| Dimethyl phthalate | - | 20 | - | 20 |
| Tromethamine | 15 | 15 | - | - |
| Arginine | - | - | 15 | 15 |
| Talc | 10 | 10 | 10 | 10 |
| Titanium dioxide | 5 | 5 | 5 | 5 |

**TABLE VI**

| **Component** | **23** | **24** | **25** | **26** |
|---|---|---|---|---|
| Didanosine | 400 | 400 | 400 | 400 |
| Eudragit^{®} L-30 D55 | 280 | 280 | 280 | 280 |
| Triethyl citrate | - | 20 | - | 20 |
| Dibutyl sebacate | 20 | - | - | - |
| Dimethyl phthalate | - | 20 | - | - |
| Tromethamine | 15 | 15 | 15 | - |
| Arginine | - | - | - | 15 |
| Talc | 15 | 15 | 15 | 15 |
| Titanium dioxide | 5 | - | - | - |

### Example 27. Capsules

The enteric coated crystals prepared in Example 1 were sieved, and those which had a size greater than 100 microns were selected.

The solid composition of the invention was metered into a capsule-forming machine at 800 mg/capsule, and capsules which contained 400 mg of didanosine in the form of coated crystals were thus prepared.

Capsules with the compositions of the invention prepared in Examples 2 to 26 were prepared in the same way, using the amount of composition necessary for obtaining capsules with a content of 400 mg of didanosine.

### Example 28. Tablets

The enteric coated crystals prepared in Example 18 were sieved, and those which had a size greater than 100 microns were selected.

Said crystals were used to prepare tablets which contained 400 mg of active ingredient according to the process described below.

730 g of the coated didanosine crystals were placed in a V-shaped mixer and 15 g of cornstarch (shock absorbing agent), 50 g of gum arabic powder (shock absorbing agent), 10 g of croscarmellose sodium (disintegrant agent) and 5 g of magnesium stearate (lubricating agent) were added.

All the components were mixed until achieving a good degree of homogenization and the mixture was compressed in a rotary compressing machine.

Tablets weighing 810 mg which contained 400 mg of didanosine in the form of enteric coated capsules were obtained.

Tablets with the compositions of the invention prepared in Examples 1 to 26 were prepared in the same way, using the amount of composition necessary for obtaining tablets with a content of 400 mg of didanosine.

### Example 29. Stability test

10 g of the composition of the invention prepared in Example 18 were weighed in a glass capsule and were placed in a climatic chamber at a temperature of 25°C and a under a humidity of 60%.

10 g of the enteric coated matrix beadlets present in the hard gelatin capsules of the medicinal product VIDEX were weighed in another glass capsule and were also placed in the climatic chamber.

Both capsules were kept for 6 months in the described conditions, and the results included in table VII were obtained:

**TABLE VII**

| Time | VIDEX | | | Example 18 | | |
|---|---|---|---|---|---|---|
| | Didanosine (%) | Impurities (%) | Impurities (% Δ) | Didanosine (%) | Impurities (%) | Impurities (% Δ) |
| 0 | 99.85 | 0.43 | - | 99.61 | 0.52 | - |
| 3 months | 99.72 | 0.41 | -4.6 | 99.77 | 0.52 | 0 |
| 6 months | 99.06 | 0.50 | +16.3 | 99.34 | 0.56 | +7.7 |

The didanosine and impurity content was analyzed after 3 months and after 6 months according to a spectrophotometric process, for example like the one described in the monograph on didanosine prepared by the World Health Organization for its inclusion in The International Pharmacopoeia, published in February 2005.

The composition of the invention contained 99.34% of didanosine after 6 months of stability, and said value is in accordance with the provisions of said monograph requiring a value comprised between 98.5% and 101.0%.

With regard to the generation of impurities, it has been observed that in the stability test the composition of the invention generates a substantially smaller amount of impurities than said matrix beadlets.

After 6 months, the impurity content in the composition of the invention has increased by 7.7.%, whereas in the matrix beadlets it has increased by 16.3%.

### Example 30. Gastro-resistance test

The gastro-resistance test has been conducted with capsules which contained the composition of the invention prepared in Example 18 according to the process described in the US Pharmacopoeia, using apparatus 1 or apparatus 2 and at a speed of 100 rpm at pH 1.5, corresponding to the gastric pH, and at pH 6.8 of the phosphate buffer solution, corresponding to the pH of the intestine.

The gastro-resistance of the hard gelatin capsules of the medicinal product VIDEX has been determined in a parallel test.

Samples have been analyzed periodically, and the didanosine content was analyzed spectrophotometrically following, for example, the process described in the mentioned monograph of the World Health Organization.

Table VIII shows the didanosine content analyzed after 2 hours for each of the pH values:

**TABLE VIII**

| pH | VIDEX Didanosine (%) | Example 18 Didanosine (%) |
|---|---|---|
| 1.5 | 0 | 0 |
| 6.8 | 93.2 | 91.1 |

It is observed that after two hours in acid medium, the percentage of didanosine released in both samples is nil, whereas in a medium buffered at pH 6.8, a percentage of didanosine greater than 90% is released in both cases, 91% in the case of the composition of the invention and 93% in the case of the matrix beadlets.

Therefore, the composition of the invention comprising enteric coated didanosine crystals has a behavior substantially equivalent to that of the medicinal product VIDEX.

## Claims

1. A solid pharmaceutical composition of didanosine **characterized in that** it comprises:
a) a core consisting of didanosine crystals with an average particle size comprised between 50 and 500 microns, and
b) an enteric layer on said core comprising an enteric polymer.

2. The composition according to claim 1, **characterized in that** the average size of the didanosine crystals is comprised between 100 and 350 microns.

3. The composition according to claim 2, **characterized in that** the average size of the didanosine crystals is comprised between 200 and 250 microns.

4. The composition according to any of claims 1 to 3, **characterized in that** the enteric polymer is selected from the group consisting of: cellulose acetate phthalate, hydroxypropyl methylcellulose phthalate, polyvinyl acetate phthalate and copolymers of methacrylic acid and acrylic or methacrylic acid esters.

5. The composition according to claim 4, **characterized in that** the enteric polymer is a copolymer of methacrylic acid and acrylic or methacrylic acid esters.

6. The composition according to claim 5, **characterized in that** the enteric polymer is selected from the group consisting of copolymers of methacrylic acid and ethyl acrylate in a 1:1 molar ratio and copolymers of methacrylic acid and methyl methacrylate in a 1:1 molar ratio.

7. The composition according to any of claims 1 to 6, **characterized in that** the enteric layer is formed by a mixture of a copolymer of methacrylic acid and ethyl acrylate in a 1:1 molar proportion and a copolymer of ethyl acrylate and methyl methacrylate.

8. The composition according to any of claims 1 to 7, **characterized in that** the amount of enteric polymer is comprised between 10% and 50% by weight over the weight of didanosine.

9. The composition according to claim 8, **characterized in that** the amount of enteric polymer is comprised between 15% and 35%.

10. The composition according to claim 9, **characterized in that** the amount of enteric polymer is comprised between 28% and 22%.

11. The composition according to any of claims 1 to 10, **characterized in that** the enteric layer further contains auxiliary agents selected from the group consisting of plasticizers, alkaline compounds, pigments and/or colorants.

12. The composition according to claim 11, **characterized in that** the plasticizer is selected from the group consisting of polyethylene glycol, propylene glycol, glycerin, simethicone, triacetin, citric acid esters, sebacic acid esters, phthalic acid esters, medium chain fatty acids mono-, di- and triglycerides, castor oil, long chain fatty acids or mixtures thereof.

13. The composition according to claim 12, **characterized in that** the plasticizer is selected from the group consisting of triethyl citrate, dimethyl phthalate and dibutyl sebacate.

14. The composition according to claim 13, **characterized in that** the plasticizer is triethyl citrate.

15. The composition according to any of claims 11 to 14, **characterized in that** the plasticizer content is comprised between 3% and 30% by weight over the weight of the enteric polymer.

16. The composition according to claim 15, **characterized in that** the plasticizer content is comprised between 10% and 25%.

17. The composition according to claim 16, **characterized in that** the alkaline compound is selected from the group consisting of sodium hydroxide, tromethamine, arginine and lysine.

18. The composition according to claim 17, **characterized in that** the alkaline compound is selected from the group consisting of sodium hydroxide and tromethamine.

19. The composition according to any of claims 1 to 18, **characterized in that** it comprises an insulating layer between the didanosine crystals and the enteric layer, wherein the insulating layer comprises a film-forming agent.

20. The composition according to claim 19, **characterized in that** the film-forming agent is selected from the group consisting of polyethylene glycol, polyvinyl alcohol, methylcellulose, hydroxypropyl methylcellulose, hydroxypropyl cellulose, ethyl cellulose, polyvinylpyrrolidone and/or mixtures thereof.

21. The composition according to claim 20, **characterized in** the film-forming agent is selected from the group consisting of hydroxypropyl methylcellulose, hydroxypropyl cellulose and ethyl cellulose.

22. The composition according to claim 21, **characterized in that** the film-forming agent is hydroxypropyl methylcellulose.

23. The composition according to any of claims 19 to 22, **characterized in that** the insulating layer comprises an alkaline compound.

24. The composition according to claim 23, **characterized in that** the alkaline compound is selected from the group consisting of magnesium oxide, magnesium hydroxide, magnesium carbonate, sodium hydroxide, sodium carbonate, tromethamine, triethanolamine, arginine, lysine and/or mixtures thereof.

25. The composition according to claim 24, **characterized in that** the alkaline compound is selected from the group consisting of tromethamine, arginine, lysine, magnesium carbonate and/ mixtures thereof.

26. A process for preparing the composition of claims 1 to 25, **characterized in that** it comprises the following steps:
a) placing the didanosine crystals in a fluid bed processor and keeping them at a temperature comprised between 20°C and 30°C,
b) applying an enteric layer comprising an enteric polymer, and
c) drying the didanosine crystals coated with the enteric layer.

27. The process according to claim 26, **characterized in that** it comprises the following steps:
a) placing the didanosine crystals in a fluid bed processor and keeping them at a temperature comprised between 20°C and 30°C,
b) applying an insulating layer comprising a film-forming agent,
c) drying the didanosine crystals coated with the insulating layer,
d) applying an enteric layer comprising an enteric polymer, and
e) drying the didanosine crystals coated with the enteric layer.

28. A solid pharmaceutical composition obtainable by means of the process of claims 26 or 27.

29. A use of the composition of any of claims 1 to 25 or 28 for preparing solid dosage forms of didanosine for their oral administration.

30. The use of claim 29, **characterized in that** the solid dosage forms are hard gelatin capsules or tablets.

31. A hard gelatin capsule **characterized in that** it comprises an amount of the composition of any of claims 1 to 25 or 28 sufficient for providing an effective unit dose of didanosine.

32. The capsule according to claim 31, **characterized in that** it comprises an effective unit dose of didanosine comprised between 100 and 600 mg.

33. The capsule according to claim 31, **characterized in that** it comprises a composition comprising:
- a core consisting of didanosine crystals, and
- an enteric layer comprising a copolymer of methacrylic acid and ethyl acrylate, triethyl citrate and tromethamine.

34. The capsule according to claim 33, **characterized in that** it comprises a composition comprising:
- a core consisting of didanosine crystals,
- an insulating layer comprising hydroxypropyl methylcellulose, tromethamine and magnesium carbonate, and
- an enteric layer comprising a copolymer of methacrylic acid and ethyl acrylate, triethyl citrate and tromethamine.

35. A tablet **characterized in that** it comprises an amount of the composition of any of claims 1 to 25 or 28 sufficient for providing an effective unit dose of didanosine and at least one auxiliary agent.

36. The tablet according to claim 35, **characterized in that** it comprises an effective unit dose of didanosine comprised between 100 and 600 mg.

37. The tablet according to claim 35, **characterized in that** it comprises a composition comprising:
- a core consisting of didanosine crystals, and
- an enteric layer comprising a copolymer of methacrylic acid and ethyl acrylate, triethyl citrate, and tromethamine.

38. The tablet according to claim 37, **characterized in that** it comprises a composition comprising:
- a core consisting of didanosine crystals,
- an insulating layer comprising hydroxypropyl methylcellulose, tromethamine and magnesium carbonate, and
- an enteric layer comprising a copolymer of methacrylic acid and ethyl acrylate, triethyl citrate and tromethamine.
